# EUROPÄISCHE PATENTSCHRIFT

(11) **EP 0 563 611 B1**
(45) Veröffentlichungstag und Bekanntmachung des Hinweises auf die Patenterteilung: **23.04.1997**
(21) Anmeldenummer: 93103479.7
(22) Anmeldetag: 04.03.1993
(51) Int. Cl.: C07C 29/17, C07C 35/12

(54) **Verfahren zur Herstellung von d,1-Menthol**
Method of preparation of d,1-menthol
Procédé pour la préparation de d,1-menthol

(30) Priorität: 17.03.1992 DE 4208443
(43) Veröffentlichungstag der Anmeldung: 06.10.1993
(73) Patentinhaber: BAYER AG, 51368 Leverkusen (DE)
(72) Erfinder: Immel, Otto, Dr., W-4150 Krefeld (DE); Darsow, Gerhard, Dr., W-4150 Krefeld (DE); Buysch, Hans-Josef, Dr., W-4150 Krefeld (DE)

(56) Entgegenhaltungen:
- EP-A- 0 351 661
- GB-A- 285 403
- US-A- 4 058 571
- CHEMICAL ABSTRACTS, vol. 105, no. 21, 1986, Columbus, Ohio, US; abstract no. 191414, A. TUNGLER et al: "Stereoselective hydrogenation of thymol", Seite 754, Spalte 2

## Beschreibung

Die Erfindung betrifft ein kontinuierliches Verfahren zur Herstellung von d,l-Menthol in Gegenwart eines Festbett-Katalysators, der Palladium, Ruthenium oder Rhodium oder ein Gemisch aus diesen Elementen als Aktivbestandteile enthält, ferner Promotoren enthält und dessen Träger mit einem Seltenen Erdmetall und Mangan dotiert ist.

Das hergestellte d,l-Menthol kann entweder in der racemischen Form oder nach einer Racematspaltung in Form seines l- oder d-Antipoden in der Riechstoff-, Geschmacksstoff-und Arzneimittelindustrie eingesetzt werden.

Es ist bekannt, d,l-Menthol durch diskontinuierliche katalytische Hydrierung von Thymol und anderen Verbindungen herzustellen, die das Kohlenstoffgerüst des Menthols mit wenigstens einer Doppelbindung in 4-Stellung besitzen und durch Sauerstoff substituiert sind, Ebenso ist die Herstellung von d,l-Menthol durch Racemisierung und/oder Umlagerung von d-Menthol und den optisch aktiven oder inaktiven Stereoisomeren des Menthols bekannt, die beispielsweise durch Erhitzen dieser Verbindungen mit Wasserstoff in Gegenwart eines Hydrierungskatalysators erfolgen kann. Dabei erhält man im allgemeinen unabhängig vom Ausgangsmaterial sowohl bei der katalytischen Hydrierung als auch bei der Racemisierung infolge der bei solchen Verfahren erfolgenden Epimerisierung in jedem Fall ein Rohprodukt, das d,l-Menthol, d,l-Neomenthol, d,l-Isomenthol und d, l-Neoisomenthol im Verhältnis von etwa 6:3:1:0,2 Gewichtsteilen entsprechend einer Gleichgewichtseinstellung enthält. Neben den vorgenannten Stereoisomeren entstehen jedoch zusätzlich Nebenprodukte, wie Menthon und Isomenthon, sowie weiterhin Menthenon und Kohlenwasserstoffe, wie Menthene und Menthane. Die Bildung dieser Nebenprodukte, die sich nur schwer oder gar nicht zu d,l-Menthol reduzieren und/oder umlagern lassen, bedingt die erheblichen Nachteile von Verfahren des Standes der Technik der geschilderten Art; beispielsweise fallen bei dem in US 2 843 636 beschriebenen Verfahren etwa 5 % nicht wieder verwendbare Kohlenwasserstoffe an.

Die geschilderten Nachteile fallen insbesondere dann ins Gewicht, wenn man versucht, diese nur für die diskontinuierliche Arbeitsweise beschriebenen Verfahren kontinuierlich durchzuführen, indem man aus dem Produktstrom mittels physikalischer Trennverfahren das d,l-Menthol abtrennt und die Nebenprodukte im Kreis führt. Hierbei reichern sich diese unerwünschten Nebenprodukte im Kreisstrom sehr schnell derart an, daß eine hierauf basierende kontinuierliche Verfahrensweise unwirtschaftlich wird. Aus DE-AS 23 14 813 ist bekannt, daß man d,l-Menthol durch katalytische Hydrierung von Verbindungen, die das Kohlenstoffgerüst des Menthols mit wenigstens einer Doppelbindung besitzen und in 3-Stellung zur Methylgruppe durch Sauerstoff substituiert sind, und/oder durch Umlagerung von optisch aktiven oder inaktiven Stereoisomeren des Menthols bei erhöhter Temperatur und unter Druck herstellen kann, wenn man unter Verwendung eines in einem Festbett angeordneten Kobalt-Katalysators mit 10 bis 40 Gew.-% Mangangehalt, bezogen auf die Gesamtmenge an Kobalt und Mangan, kontinuierlich hydriert, wobei die Wasserstoffmenge je Mol Einsatzmaterial mindestens das 10-fache der zur Hydrierung eines Benzolkerns erforderlichen Menge beträgt; hierbei wird im Temperaturbereich von 170 bis 220°C und unter einem Druck von mindestens 25 bar gearbeitet. Bevorzugt findet ein Kobalt-Katalysator mit einem Gehalt von 15 bis 30, insbesondere 20 bis 25 Gew.-% Mangan Verwendung.

Die Verwendung des Kobalt-Mangan-Katalysators gemäß der genannten DE-AS 23 14 813 ist jedoch technisch aufwendig und umweltbelastend. In der genannten DE-AS finden sich darüber hinaus keine Angaben über die mechanische Stabilität und Lebensdauer dieses Katalysators, die für eine technische Produktion von d,l-Menthol von maßgebender Bedeutung sind.

Es bestand daher der Wunsch, einen einfach herzustellenden langlebigen und ökologisch unbedenklichen Katalysator zu finden, der es gestattet, das grundsätzlich bekannte Verfahren zur Herstellung von d,l-Menthol kontinuierlich in technischem Umfang durchzuführen, Im Sinne der Erfindung gelingt dies mit einem Festbett-Katalysator, der als Aktivbestandteile Palladium, Ruthenium, Rhodium oder ein Gemisch aus diesen Elementen enthält, weiterhin Alkalimetallhydroxide und/oder Alkalimetallsulfate als Promotoren enthält und dessen Träger durch ein seltenes Erdmetall und Mangan dotiert ist.

Die Erfindung betrifft ein Verfahren zur Herstellung von d,l-Menthol durch katalytische Hydrierung von Verbindungen, die das Kohlenstoffgerüst des Menthols mit wenigstens einer Doppelbindung besitzen und in 3-Stellung zur Methylgruppe durch Sauerstoff substituiert sind, bei gleichzeitiger Umlagerung von optisch aktiven oder inaktiven Stereoisomeren des Menthols, wobei die Wasserstoffmenge je Mol Ausgangsmaterial mindestens das 5-fache der zur Hydrierung eines Benzolkerns erforderlichen Menge beträgt und wobei im Temperaturbereich von 160 bis 220°C unter einem Druck von mindestens 25 bar gearbeitet wird, das dadurch gekennzeichnet ist, daß man die Hydrierung kontinuierlich an einem Festbett-Katalysator durchführt, der als Aktivbestandteile Palladium, Ruthenium, Rhodium oder ein Gemisch aus diesen Elementen enthält, der als Promotoren eines oder mehrere Alkalimetallhydroxide und/oder Alkalimetallsulfate enthält und dessen Träger ein oder mehrere Seltene Erdmetalle und Mangan enthält.

Als Träger kommen Aluminium enthaltende Materialien in Frage, wie Aluminiumoxide, Aluminiumspinelle, Aluminiumphosphat oder Alumosilikate, bevorzugt Aluminiumoxide, besonders bevorzugt α- oder γ-Al₂O₃. In ganz besonders bevorzugter Weise wird γ-Al₂O₃ als Träger eingesetzt.

Der Träger ist mit einer oder mehreren Verbindungen von Seltenen Erdmetallen und des Mangans dotiert. Der Gehalt an Seltenen Erdmetallen und Mangan beträgt zusammen 0,05 bis 8 Gew.-%, bevorzugt 0,2 bis 5 Gew.-%, bezogen auf das Gesamtgewicht des Katalysators. Das Gewichtsverhältnis von Seltenen Erdmetallen zu Mangan beträgt 5:1 bis 1:5, bevorzugt 2:1 bis 1:2. Als Seltene Erdmetalle werden die Elemente der III. Nebengruppe des Periodensystems (Mendelejew), wie Scandium, Yttrium, Lanthan und die Lanthaniden verstanden. In bevorzugter Weise werden Yttrium, Lanthan, Cer, Praseodym, Neodym und Dysprosium, in besonders bevorzugter Weise Cer und Lanthan und in ganz besonders bevorzugter Weise Cer verstanden, Die Seltenen Erdmetalle kommen vielfach miteinander vergesellschaftet vor. Das ganz besonders bevorzugte Cer kann beispielsweise mit Lanthan, Praseodym, Neodym, Dysprosium oder mit Yttrium oder mit mehreren von ihnen vergesellschaftet sein. Eine solche Vergesellschaftung ist dem Fachmann im übrigen für alle genannten Seltenen Erdmetalle geläufig.

Die als Aktivbestandteile wirkenden Edelmetalle Palladium, Ruthenium, Rhodium oder ein Gemisch mehrerer von ihnen liegen in einer Gesamtmenge von 0,05 bis 5, bevorzugt 0,05 bis 4, besonders bevorzugt 0,1 bis 3 Gew.-%, bezogen auf das Gesamtgewicht des Katalysators, vor. Unter den genannten Edelmetallen sind Palladium oder eine Palladiumkombination mit Ruthenium, Rhodium oder Ruthenium und Rhodium bevorzugt. Für den Fall einer Kombination beträgt der Anteil des Palladiums 10-95 Gew.-%, bevorzugt 15-80 Gew.-%, besonders bevorzugt 20-70 Gew.-%, bezogen auf das Gesamtgewicht von Palladium und anderen Edelmetallen. In der folgenden Beschreibung steht jedoch die Bezeichnung Palladium stets auch für die beiden anderen Edelmetalle oder für beliebige Kombinationen aus den drei genannten Edelmetallen.

Zur Herstellung der erfindungsgemäß einzusetzenden Katalysatoren kann so vorgegangen werden, daß auf den Träger, in bevorzugter Weise auf ein Aluminiumoxid, in Form von Strangpreßlingen, Pillen oder Kugeln mit Durchmessern von etwa 2 bis 10 mm Verbindungen der Seltenen Erdmetalle und des Mangans aufgebracht werden. Der so dotierte Träger wird nach dem Trocknen auf 200 bis 450°C erhitzt und anschließend mit einer Lösung eines Palladiumsalzes (oder eines Salzes der anderen Edelmetalle oder von Salzen von Edelmetallgemischen im Sinne des oben Gesagten) getränkt oder besprüht, wonach sich eine erneute Trocknungsphase anschließt, Das Aufbringen von Verbindungen der Seltenen Erdmetalle und des Mangans auf den Katalysatorträger kann beispielsweise durch bloßes Tränken oder Sprühen mit geeigneten Salzen der Seltenen Erdmetalle und des Mangans erfolgen. Das Aufbringen von Verbindungen der Seltenen Erdmetalle und des Mangans kann jedoch auch durch gemeinsames Ausfällen eines Seltenenerd/Mangan-Hydroxidgemisches aus Seltenerd- und Mangansalzen auf dem Träger mit Alkalilauge oder Ammoniak und gegebenenfalls anschließendes Auswaschen der löslichen Anteile mit Waser erfolgen. Als Seltenerd- und Mangansalze kommen insbesondere die Sulfate, Chloride, Acetate und/oder Nitrate der genannten Elemente in Betracht. Nach dem Aufbringen der Seltenerd- und Mangansalze und gegebenenfalls nach der beschriebenen Ausfällung und der darauf erfolgenden Auswaschung wasserlöslicher Verbindungen wird der so behandelte Träger zunächst getrocknet, bevor er auf die genannte höhere Temperatur, etwa auf 200 bis 450°C` bevorzugt 250 bis 430°C, erhitzt wird. Dieses Erhitzen erfolgt in einer Zeit von 1 bis 120 Stunden. Während dieser Zeit kann die Temperatur im angegebenen Bereich von niederen auf höhere Werte erhöht werden.

Nach der beschriebenen Temperung wird der so dotierte Katalysatorträger mit einer Palladium (Edelmetalle) enthaltenden Lösung getränkt. Hierbei kann so vorgegangen werden, daß das Palladium (die Edelmetalle) beispielsweise in Form wäßriger Lösungen des Chlorids, Nitrats, Acetats oder eines anderen geeigneten Salzes auf den Träger aufgetränkt oder aufgesprüht wird, gefolgt von einer Trocknung. Gegebenenfalls können die Palladiumsalze, beispielsweise Palladium-acetat auch in organischen Lösungsmitteln, wie Methanol, Methylenchlorid, Acetonitril oder Dioxan, in Lösung gebracht und in dieser Weise aufgetränkt werden. Man kann jedoch auch vor der Trocknung den mit Palladiumsalzen getränkten Träger mit einer Lösung der obengenannten basischen Verbindungen behandeln, wobei das Palladium als Oxid oder Hydroxid ausfällt. Auch nach dieser Variante der Aufbringung von Palladium schließt sich eine Trocknung an. Man kann jedoch auch den mit Verbindungen der Seltenen Erdmetalle und des Mangans beaufschlagten Katalysatorträger zunächst mit der Lösung einer der genannten basischen Verbindungen tränken, anschließend trocknen und auf den so vorbehandelten, basisch eingestellten Katalysatorträger Lösungen von Palladiumsalzen auftragen, wobei im Moment der Tränkung auch die Ausfällung des Palladiums in Form seines Oxids oder Hydroxids erfolgt.

Der einzusetzende Katalysator enthalt weiterhin 1 bis 6 Gew.-%, bevorzugt 2 bis 5 Gew.-%, bezogen auf das Gesamtgewicht des Katalysators an einem oder mehreren Alkalihydroxiden und/oder 1 bis 6 Gew.-%, bevorzugt 2 bis 5 Gew.-%, ebenfalls bezogen auf das Gesamtgewicht des Katalysators, an einem oder mehreren Alkalimetallsulfaten. Alkalimetallhydroxide sind Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid oder Kaliumhydroxid. Alkalimetallsulfate sind Lithiumsulfat, Natriumsulfat, Kaliumsulfat, Rubidiumsulfat, Caesiumsulfat, bevorzugt Lithiumsulfat, Natriumsulfat, Kaliumsulfat, besonders bevorzugt Natriumsulfat oder Kaliumsulfat. Auch nach dem Auftragen der Alkalimetallhydroxide und/oder Alkalimetallsulfate schließt sich eine Trocknung, im allgemeinen bei 100 bis 140°C bei vermindertem bis normalem Druck (1 bis 1000 mbar, bevorzugt 10 bis 500 mbar, beispielsweise im Wasserstrahlvakuum) an.

In bevorzugter Weise trägt der Katalysator als Promotoren sowohl Hydroxide als auch Sulfate im Rahmen der genannten Mengen.

Das Auftränken des Palladiums (der Edelmetalle) bzw. der Alkalihydroxide und/oder Alkalimetallsulfate erfolgt getrennt. Hierbei kann so vorgegangen werden, daß zunächst das Palladium in der oben beschriebenen Weise aufgetränkt wird, wobei nach einer Trocknung eine weitere Tränkung mit Alkalimetallhydroxiden und Alkalimetallsulfaten erfolgt. Bei dieser Behandlung wird das Palladium in Form seines Oxids oder Hydroxids ausgefällt. Die Alkalimetallhydroxide und die Alkalimetallsulfate können getrennt oder gemeinsam aufgetränkt werden. Man kann jedoch auch den Träger zunächst mit einer Alkalimetallhydroxidlösung tränken, anschließend trocknen und auf den so vorbehandelten basisch eingestellten Katalysatorträger Salze des Palladiums auftragen, wobei im Moment der Tränkung auch die Ausfällung des Palladiums in Form des Oxids oder Hydroxids erfolgt. Das Auftragen der Alkalimetallsulfate kann bei dieser Variante gemeinsam mit dem Alkalimetallhydroxid oder als abschließende Tränkung nach dem Auftragen des Palladiums erfolgen. In der bereits beschriebenen Weise wird nach jedem Auftragen getrocknet.

Statt einer Tränkung mit den genannten Salzlösungen kann auch ein Besprühen erfolgen. Die hierzu erforderlichen Arbeitsgeräte und die Einstellung der gewünschten Beaufschlagung durch Wahl der Menge und Konzentration der Lösungen der genannten Stoffe sind dem Fachmann grundsätzlich bekannt.

Ein in der genannten Weise hergestellter Träger steht nach der letzten Trocknungsphase grundsätzlich für den erfindungsgemäßen Einsatz zur Verfügung. In bevorzugter Weise wird er jedoch vor seinem Einsatz, besonders bevorzugt nach Anordnung im Hydrierreaktor, durch Behandlung mit Wasserstoff bei einer Temperatur von 150 bis 400°C aktiviert.

Die für das erfindungsgemäße Verfahren Verwendung findenden Ausgangsverbindungen sind bekannt (Ullmanns Encyclopädie der technischen Chemie, 3. Auflage, Band 17, München 1966, S. 24/25; US 2 843 636). Beispielsweise seien genannt: Menthon, Isomenthol, d- und l-Menthol, d- und l-Neomenthol, d- und l-Isomenthol, d,l-Neomenthol, d,l-Isomenthol, Thymol, bevorzugt Thymol. Diese Verbindungen können sowohl einzeln als auch in beliebigen Gemischen untereinander verwendet werden.

Das erfindungsgemäße Verfahren wird mit dem im Festbett angeordneten Katalysator in der Gas- bzw. Rieselphase durchgeführt. Es wird bei Wasserstoffüberschuß gearbeitet; die H₂-Menge beträgt das 5 bis 20-fache der Menge, die für die Hydrierung eines Benzolkerns erforderlich ist. Es wird bei 160 bis 220°C, bevorzugt bei 160 bis 210°C und bei einem Druck von über 25 bar, bevorzugt über 100 bar, besonders bevorzugt über 200 bar gearbeitet. Die Obergrenze des zur Anwendung gelangenden Drucks ergibt sich sowohl aus technischen als auch aus wirtschaftlichen Überlegungen und liegt bei 500 bar, bevorzugt bei 200-350 bar.

Die beim erfindungsgemäßen Verfahren erfolgende Hydrierung und Racemisierung sowie Isomerisierung laufen überraschenderweise unter den zur Anwendung gelangenden Reaktionsbedingungen so milde ab, daß die Bildung unverwertbarer Nebenprodukte, wie unerwünschter Kohlenwasserstoffe, gegenüber den bekannten Verfahren stark vermindert wird.

Die Anordnung des erfindungsgemäß einzusetzenden Katalysators kann in verschiedenen, dem Fachmann für solche Zwecke grundsätzlich bekannten Apparaten erfolgen. In vorteilhafter Weise wird das erfindungsgemäße Verfahren in Röhrenreaktoren mit einer oder mehreren Röhren durchgeführt. Die Reaktionsrohre können Längen von beispielsweise 2 bis 20 m und innere Durchmesser von 20 bis 800 mm besitzen. Die Katalysatoren haben beispielsweise Korngrößen von 2 bis 10 mm.

Das erfindungsgemäße Verfahren kann mit oder ohne Lösungsmittel durchgeführt werden. Geeignete, unter den Reaktionsbedingungen inerte Lösungsmittel sind beispielsweise Methanol, Ethanol, Isopropanol.

Das erhaltene Reaktionsgemisch enthält einen so hohen Gehalt an d,l-Menthol, daß es durch einfache Destillation auf dieses gewünschte Produkt aufgearbeitet werden kann. Man erhält mit Hilfe des erfindungsgemäßen Verfahrens nicht nur ausgezeichnete Ergebnisse bei der Hydrierung von Thymol, sondern auch ausgezeichnete Ausbeuten bei der Umsetzung anderer, weiter obengenannter Ausgangsverbindungen.

Nach der destillativen Abtrennung des gewünschten d,l-Menthols kann der Destillationsvorlauf mit dem Destillationssumpf unter Zusatz von frischem Ausgangsprodukt, beispielsweise unter Zusatz von 30 bis 80 Gew.-% Thymol, bezogen auf diesen Destillationsrückstand, wieder in die Reaktion zurückgeführt werden. Die zugefügte Menge an Ausgangsmaterial entspricht in vorteilhafter Weise dem destillativ entnommenen d,l-Menthol. Auch der im erfindungsgemäßen Verfahren nicht verbrauchte Wasserstoff kann im Kreis geführt werden.

### Bespiel 1

200 g eines handelsüblichen γ-Al₂O₃ mit einer spezifischen Oberfläche von 350 m² /g und einem Kugeldurchmesser von 2 bis 6 mm wurden mit einer Lösung getränkt, die aus 12,4 g Ce(NO₃)₃ · 6 H₂O, 18,28 g Mn(NO₃)₂ · 4 H₂O und 75 g Wasser hergestellt worden war. Das getränkte Al₂O₃ wurde im Wasserstrahlvakuum 18 Stunden bei 120°C getrocknet und anschließend 3 Stunden lang bei 400°C getempert.

100 g des so hergestellten Katalysatorträgers wurden mit einer Lösung getränkt, die aus 4,16 g Pd-Acetat und 30 g Dioxan hergestellt worden war. Der Katalysator wurde 18 Stunden bei 100°C getrocknet. Bei der Tränkung wurden 2 Gew.-% Pd, bezogen auf das Gesamtgewicht des Katalysators, aufgebracht.

100 g des so hergestellten Katalysators wurden zusätzlich mit einer Lösung getränkt, die aus 3 g K₂SO₄ und 30 g Wasser hergestellt worden war. Der mit Pd (2 %) getränkte Katalysator wurde 18 Stunden bei 100°C getrocknet.

60 ml (51,3 g) des so hergestellten Katalysators wurden für die kontinuierliche Thymol-Hydrierung in ein Druckrohr gefüllt, und es wurde nach der in Beispiel 1 beschriebenen Weise verfahren. Zunächst wurde der Katalysator wieder bei 250°C und 270 bar aktiviert, bevor mit der kontinuierlichen Hydrierung von Thymol begonnen wurde.

Der Thymol-Durchsatz entsprach einer Katalysatorbelastung von 0,2 bis 0,3 g/ml · Kat. x h. Aus dem Druckabscheider wurden stündlich 70 bis 90 l Wasserstoff entspannt. Das Reaktionsprodukt zeigte in Abhängigkeit von der Hydriertemperatur und der Versuchsdauer folgende Zusammensetzung (laut GC-Analysen).

| Laufzeit (h) | d,l-Menthol (F-%) | d,l-Neomenthol (F-%) |
|---|---|---|
| 90 | 54,6 | 29,6 |
| 138 | 55,6 | 31,4 |
| 162 | 55,1 | 31,6 |
| 477 | 55,5 | 30,8 |
| 547 | 55,6 | 30,5 |

Im Hydrierprodukt wurde kein Thymol gefunden. Der Rest zu 100 % bestand hauptsächlich aus d,l-Isomenthol, d,l-Neoisomenthol und Menthanverbindungen.

### Beispiel 2

Ein gemäß Beispiel 1 des EP 0 351 661 B1 hergestellter Ru-Ce-Mn-Al₂O₃-Katalysator wurde zusätzlich mit Natriumhydroxid imprägniert (1,8 g NaOH/100 ml Katalysator), erneut getrocknet und zur Hydrierung von Thymol eingesetzt. Der Katalysator wurde 3 Stunden in Wasserstoffstrom bei 250°C aktiviert.

Zur Thymolhydrierung wurde ein 250 ml Schüttelautoklav verwendet, der im Innern mit einem zentral gelagerten, fest mit dem Autoklaven verbundenen Siebkorb ausgestattet war, in den 25 ml (20,1 g) des aktivierten Katalysators gefüllt wurde. Mit dieser Katalysatorfüllung wurden 50 g Thymol unter einem Wasserstoffdruck von 240 bis 300 bar bei 180°C hydriert. Die Hydrierzeit betrug 6,2 Stunden, Das Hydrierprodukt enthielt laut Gaschromatographie 56,2 F % d,l-Menthol.

## Patentansprüche

1. Verfahren zur Herstellung von d,l-Menthol durch katalytische Hydrierung aromatischer oder teilhydrierter cyclischer Verbindungen, die das Kohlenstoffgerüst des Menthols mit wenigstens einer Doppelbindung besitzen und in 3-Stellung zur Methylgruppe durch Sauerstoff substituiert sind, bei gleichzeitiger Umlagerung von optisch aktiven oder inaktiven Stereoisomeren des Menthols, wobei die Wasserstoffmenge je Mol Ausgangsmaterial mindestens das 5- fache der zur Hydrierung eines Benzolkerns erforderlichen Menge beträgt und wobei im Temperaturbereich von 160 bis 220°C unter einem Druck von mindestens 25 bar gearbeitet wird, dadurch gekennzeichnet, daß man die Hydrierung kontinuierlich an einem Festbett-Katalysator durchführt, der als Aktivbestandteile Palladium, Ruthenium, Rhodium oder ein Gemisch aus diesen Elementen enthalt, der als Promotoren eines oder mehrere Alkalimetallhydroxide und/oder Alkalimetallsulfate enthält und dessen Träger ein oder mehrere Seltene Erdmetalle und Mangan enthält.

2. Verfahren nach Anspruch 1, dadurch gekennzeichnet, daß als Träger ein α- oder γ-Al₂O₃ zunächst mit mindestens einer Verbindung von Seltenen Erdmetallen (III. Nebengruppe des Periodensystems der Elemente nach Mendelejew) und mit mindestens einer Verbindung des Mangans behandelt wird, wobei die Menge der Seltenen Erdmetalle und des Mangans insgesamt 0,05 bis 8 Gew.-%, bevorzugt 0,2 bis 5 Gew.-%, bezogen auf das Gesamtgewicht des Katalysators, und das Gewichtsverhältnis von Seltenen Erdmetallen zu Mangan 5:1 bis 1:5, bevorzugt 2:1 bis 1:2, betragen.

3. Verfahren nach Anspruch 1, dadurch gekennzeichnet, daß der Träger mit einer solchen Menge mindestens einer Verbindung von Palladium, Ruthenium, Rhodium oder einem Gemisch mehrerer von ihnen behandelt wird, daß der Gehalt dieser Edelmetalle 0,05 bis 5 Gew.-%, bevorzugt 0,05 bis 4 Gew.-%, besonders bevorzugt 0,1 bis 3 Gew.-%, bezogen auf das Gesamtgewicht des Katalysators, beträgt.

4. Verfahren nach Anspruch 1, dadurch gekennzeichnet, daß als Seltenes Erdmetall eines oder mehrere aus der Gruppe von Yttrium, Lanthan, Cer, Praseodym, Neodym und Dysprosium, bevorzugt aus der Gruppe von Cer und Lanthan, eingesetzt wird (werden) und besonders bevorzugt Cer eingesetzt wird.

5. Verfahren nach Anspruch 1, dadurch gekennzeichnet, daß der Katalysator Palladium oder eine Kombination Palladium/Ruthenium, Palladium/Rhodium oder Palladium/Ruthenium/Rhodium enthält und daß für den Fall einer Kombination der Gewichtsanteil des Palladiums in der Kombination Palladium/andere Edelmetalle 10 bis 95 %, bevorzugt 15 bis 80 %, besonders bevorzugt 20 bis 70 %, bezogen auf das Gesamtgewicht aller Edelmetalle, beträgt.

6. Verfahren nach Anspruch 1, dadurch gekennzeichnet, daß der Anteil der Promotoren 1 bis 6 Gew.-%, bevorzugt 2 bis 5 Gew.-% Hydroxide und/oder 1 bis 6 Gew.-%, bevorzugt 2 bis 5 Gew.-% Sulfate beträgt, wobei alle Gewichtsprozente auf das Gesamtgewicht des Katalysators bezogen sind.

7. Verfahren nach Anspruch 1, dadurch gekennzeichnet, daß bei einem Druck von 25 bis 500 bar, bevorzugt 100 bis 500 bar, besonders bevorzugt 200 bis 350 bar gearbeitet wird.

8. Verfahren nach Anspruch 1, dadurch gekennzeichnet, daß bei einer Temperatur von 160 bis 210°C gearbeitet wird.

9. Verfahren nach Anspruch 1, dadurch gekennzeichnet, daß als Ausgangsstoff Thymol eingesetzt wird.

10. Verfahren nach Anspruch 1, dadurch gekennzeichnet, daß aus dem Reaktionsprodukt der gleichzeitigen Hydrierung und Umlagerung das d,l-Menthol destillativ entnommen wird und die restlichen Reaktionsprodukte unter Zusatz von 30 bis 80 Gew.-% Thymol, bezogen auf die restlichen Reaktionsprodukte, in die Reaktion zurückgeführt werden.

## Claims

1. Process for the preparation of d,l-menthol by catalytic hydrogenation of aromatic or partly hydrogenated cyclic compounds which have the carbon skeleton of menthol with at least one double bond and are substituted by oxygen in the 3-position relative to the methyl group, with simultaneous rearrangement of optically active or inactive stereoisomers of menthol, the amount of hydrogen per mole of starting material being at least 5 times the amount required for hydrogenation of a benzene nucleus and the reaction being carried out in the temperature range of 160 to 220°C under a pressure of at least 25 bar, characterised in that the hydrogenation is carried out continuously on a fixed bed catalyst which comprises, as the active constituents, palladium, ruthenium, rhodium or a mixture of these elements, and which comprises, as promoters, one or more alkali metal hydroxides and/or alkali metal sulphates, and the support of which comprises one or more rare earth metals and manganese.

2. Process according to Claim 1, characterised in that, as the support, an α- or γ-Al₂O₃ is first treated with at least one compound of rare earth metals (sub-group III of the Periodic Table of the Elements according to Mendeleev) and with at least one compound of manganese, the total amount of rare earth metals and of manganese being 0.05 to 8% by weight, preferably 0.2 to 5% by weight, based on the total weight of the catalyst, and the weight ratio of rare earth metals to manganese being 5:1 to 1:5, preferably 2:1 to 1:2.

3. Process according to Claim 1, characterised in that the support is treated with an amount of at least one compound of palladium, ruthenium, rhodium or of a mixture of several of them such that the content of these noble metals is 0.05 to 5% by weight, preferably 0.05 to 4% by weight, particularly preferably 0.1 to 3% by weight, based on the total weight of the catalyst.

4. Process according to Claim 1, characterised in that the rare earth metal employed is one or more from the group comprising yttrium, lanthanum, cerium, praseodymium, neodymium and dysprosium, preferably from the group comprising cerium and lanthanum, and cerium is particularly preferably employed.

5. Process according to Claim 1, characterised in that the catalyst comprises palladium or a combination of palladium/ruthenium, palladium/rhodium or palladium/ruthenium/rhodium, and in that in the case of a combination, the weight content of the palladium in the combination of palladium/other noble metals is 10 to 95%, preferably 15 to 80%, particularly preferably 20 to 70%, based on the total weight of all the noble metals.

6. Process according to Claim 1, characterised in that the content of promoters is 1 to 6% by weight, preferably 2 to 5% by weight of hydroxides and/or 1 to 6% by weight, preferably 2 to 5% by weight of sulphates, all the percentages by weight being based on the total weight of the catalyst.

7. Process according to Claim 1, characterised in that it is carried out under a pressure of 25 to 500 bar, preferably 100 to 500 bar, particularly preferably 200 to 350 bar.

8. Process according to Claim 1, characterised in that it is carried out at a temperature of 160 to 210°C.

9. Process according to Claim 1, characterised in that thymol is employed as the starting substance.

10. Process according to Claim 1, characterised in that the d,l-menthol is removed by distillation from the reaction product of the simultaneous hydrogenation and rearrangement, and the remaining reaction products are recycled into the reaction with addition of 30 to 80% by weight of thymol, based on the remaining reaction products.

## Revendications

1. Procédé pour la préparation du d,ℓ-menthol par hydrogénation catalytique de composés aromatiques ou cycliques partiellement hydrogénés qui possèdent le squelette carboné du menthol comprenant au moins une liaison double et qui sont substitués par un atome d'oxygène en position 3 par rapport au groupe méthyle, avec réarrangement simultané de stéréo-isomères optiquement actifs ou inactifs du menthol, dans lequel la quantité d'hydrogène par mole de matière de départ représente au moins le quintuple de la quantité requise pour l'hydrogénation d'un noyau benzénique et dans lequel on travaille dans le domaine de température de 160 à 220°C sous une pression d'au moins 25 bar, caractérisé en ce qu'on effectue l'hydrogénation en continu sur un catalyseur à lit fixe qui contient, comme constituants actifs, du palladium, du ruthénium, du rhodium ou un mélange de ces éléments, qui contient comme promoteurs un ou plusieurs hydroxydes de métaux alcalins et/ou sulfates de métaux alcalins et dont le support contient un ou plusieurs métaux des terres rares et du manganèse.

2. Procédé selon la revendication 1, caractérisé en ce que, à titre de support, on traite un α- ou γ-Al₂O₃ d'abord avec au moins un composé des métaux des terres rares (sous-groupe III du système périodique des éléments selon Mendelejew) et avec au moins un composé du manganèse, dans lequel la quantité des métaux des terres rares et du manganèse s'élève au total de 0,05 à 8% en poids, de préférence de 0,2 à 5% en poids rapportés au poids total du catalyseur et le rapport pondéral des métaux des terres rares au manganèse s'élève de 5:1 à 1:5, de préférence de 2:1 à 1:2.

3. Procédé selon la revendication 1, caractérisé en ce qu'on traite le support avec une quantité d'au moins un composé du palladium, du ruthénium, du rhodium ou d'un mélange de plusieurs d'entre eux telle que la teneur de ces métaux nobles s'élève de 0,05 à 5% en poids, de préférence de 0,05 à 4% en poids, de manière particulièrement préférée de 0,1 à 3% en poids rapportés au poids total du catalyseur.

4. Procédé selon la revendication 1, caractérisé en ce que, comme métal des terres rares, on met en oeuvre un ou plusieurs métaux choisis parmi le groupe comprenant l'yttrium, le lanthane, le cérium, le praséodyme, le néodyme et le dysprosium, de préférence parmi le groupe comprenant le cérium et le lanthane, et de manière particulièrement préférée, on met en oeuvre du cérium.

5. Procédé selon la revendication 1, caractérisé en ce que le catalyseur contient du palladium ou une combinaison de palladium/ruthénium, de palladium/rhodium ou de palladium/ruthénium/rhodium et en ce que, dans le cas d'une combinaison, la fraction pondérale du palladium dans la combinaison palladium/autres métaux nobles s'élève de 10 à 95%_{,} de préférence de 15 à 80%, de manière particulièrement préférée de 20 à 70% rapportés au poids total de tous les métaux nobles.

6. Procédé selon la revendication 1, caractérisé en ce que la fraction des promoteurs s'élève de 1 à 6% en poids, de préférence de 2 à 5% en poids d'hydroxydes et/ou de 1 à 6% en poids, de préférence de 2 à 5% en poids de sulfates, tous les % en poids se rapportant au poids total du catalyseur.

7. Procédé selon la revendication 1, caractérisé en ce qu'on travaille sous une pression de 25 à 500 bar, de préférence de 100 à 500 bar, de manière particulièrement préférée de 200 à 350 bar.

8. Procédé selon la revendication 1, caractérisé en ce qu'on travaille à une température de 160 à 210°C.

9. Procédé selon la revendication 1, caractérisé en ce que, comme matière de départ, on met en oeuvre le thymol.

10. Procédé selon la revendication 1, caractérisé en ce que, à partir du produit réactionnel issu de l'hydrogénation et du réarrangement simultanés, on récupère le d,ℓ-menthol par distillation et on renvoie dans la réaction les produits réactionnels résiduels avec addition de 30 à 80% en poids de thymol rapportés aux produits réactionnels résiduels.
